# EUROPÄISCHE PATENTSCHRIFT

(11) **EP 0 856 507 B1**
(45) Veröffentlichungstag und Bekanntmachung des Hinweises auf die Patenterteilung: **27.12.2000**
(21) Anmeldenummer: 98101536.5
(22) Anmeldetag: 29.01.1998
(51) Int. Cl.: C07C 51/42, C07C 59/125, C07C 209/88, C25B 3/00

(54) **Verfahren zur Reinigung von Alpha-, Beta- oder Gamma- substituierten Carbonsäuren durch Elektrodialyse**
Process for the purification of alpha-, beta- or gamma-substituted carboxylic acids by electrodialysis
Procédé de purification d'acides carboxyliques alpha-, bêta- ou gamma-substités par électrodialyse

(30) Priorität: 30.01.1997 DE 19703426
(43) Veröffentlichungstag der Anmeldung: 05.08.1998
(73) Patentinhaber: BASF AKTIENGESELLSCHAFT, 67056 Ludwigshafen (DE)
(72) Erfinder: Frede, Markus, Dr., 69214 Eppelheim (DE); Dully, Christian, 67069 Ludwigshafen (DE); Ditrich, Klaus, Dr., 67161 Gönnheim (DE); Melder, Johann-Peter, 67141 Neuhofen (DE); Weyer, Hans-Jürgen, Dr., 67240 Bobenheim-Roxheim (DE); Weitze, Achim, 38302 Wolfenbüttel (DE)
(74) Vertreter: Isenbruck, Günter, Dr.

(56) Entgegenhaltungen:
- WO-A-97/10201
- DE-A- 4 219 756
- US-A- 4 605 477
- MANI K N: "ELECTRODIALYSIS WATER SPLITTING TECHNOLOGY" JOURNAL OF MEMBRANE SCIENCE, Bd. 58, Nr. 2, 15.Mai 1991, Seiten 117-138, XP000247572

## Beschreibung

Die Erfindung betrifft ein Verfahren zur Reinigung einer α-, β- oder γ-substituierten Carbonsäure oder eines Salzes oder eines Esters davon oder eines Gemischs aus zwei oder mehr davon aus einer wäßrigen Lösung, wobei diese Lösung mindestens ein Polyol oder mindestens einen Aminoalkohol und ggf. ein Kation einer starken Base enthält und mittels Elektrodialyse behandelt wird, sowie ein Verfahren zur Spaltung eines optisch aktiven Amids und ein Verfahren zur Racematspaltung eines Gemischs aus zwei Enantiomeren eines primären oder sekundären Amins, die jeweils als eine Stufe das erfindungsgemäße Reinigungsverfahren umfassen.

Die hydrolytische Spaltung von optisch aktiven Amiden, die im Aminteil des Moleküls ein Chiralitätszentrum besitzen, ist nicht oder nur unter sehr aufwendigen Bedingungen so durchführbar, daß das Chiralitätszentrum erhalten bleibt.

Von Devant und Braun (Chem. Berichte 119, S. 2191-2207 (1986)) wird beschrieben, daß die Abspaltung von chiralen Aminen aus Acetamiden nicht ohne Zerstörung des Chiralitätszentrums möglich ist (S. 2194). Weiterhin finden die Autoren, daß zahlreiche Versuche, die Amide alkalisch oder sauer zur Carbonsäure und optisch aktivem Amin zu hydrolysieren, erfolglos geblieben sind, und daß lediglich die Umsetzung mit Distickstofftetroxid nach White (J. Am. Chem. Soc. 77, S. 6008 (1955)) zum gewünschten Ergebnis führt. Diese Umsetzung mit N₂O₄ ist jedoch aufwendig und daher nicht für technische Verfahren geeignet.

In der WO 95/08636 wird ein enzymatisches Verfahren zur Racematspaltung von optisch aktiven Aminen beschrieben, bei dem die Amine enantioselektiv mit einem Ester acyliert werden, dann das Gemisch aus acyliertem Amin (Amid) und nicht-umgesetztem Amin getrennt wird und ggf. aus dem acylierten Amin (Amid) das optisch aktive Amin durch Amidspaltung freigesetzt wird. Es werden jedoch keine Verfahrensparameter angegeben, bei denen die Amidspaltung durchgeführt werden kann.

Als Weiterführung dieses Verfahrens wird in der PCT/EP/96/03948 ein Verfahren zur Spaltung von optisch aktiven Amiden zu Carbonsäuren und optisch aktiven Aminen unter Erhalt des Chiralitätszentrums beschrieben, das dadurch gekennzeichnet ist, daß man eine Hydrolyse der Amide in Gegenwart eines Polyols oder Aminoalkohols und einem Alkali- oder Erdalkalihydroxid durchführt.

Dabei werden als Carbonsäuren, die als Hilfsreagenz zur enzymatischen Racematspaltung von optisch aktiven Aminen eingesetzt werden, vorzugsweise α-, β- oder γ-substituierte Carbonsäuren, in der Regel als wäßrige Lösung, die neben einem Salz oder einem Ester der obigen Carbonsäure mindestens ein Polyol oder mindestens einen Aminoalkohol sowie ein Alkali- oder Erdalkalihydroxid enthalten, verwendet. Diese Lösungen wurden bislang stets der Verbrennung zugeführt.

Eine destillative Auftrennung eines derartigen Gemischs ist in der Regel nicht möglich, da die Carbonsäuren bzw. deren Salze oder Ester unter Veresterung mit dem Polyol, z.B. Ethylenglykol (EG) oder Diethylenglykol (DEG), oder dem Aminoalkohol, wie z.B. Ethanolamin, Diethanolamin und Triethanolamin (TEA) und/oder unter Salzbildung (im Fall von TEA) reagieren können. Demzufolge ist zur Ausbeuteoptimierung zuvor eine möglichst vollständige Abtrennung der neben der Carbonsäure bzw. des Salzes oder Esters davon vorhandenen, oben genannten Komponenten erforderlich. Destillative Aufarbeitung ist wegen der hohen Siedepunkte der oben genannten Verbindungen im allgemeinen schwierig bzw. technisch sehr aufwendig. Ferner ist zu beachten, daß z.B. Ethylenglykol einen ähnlichen Siedepunkt wie zahlreiche der hier in Rede stehenden Carbonsäuren, wie z.B. Methoxyessigsäure, aufweist, was wiederum die destillative Abtrennung der Säure aus obigem Gemisch erschwert.

Somit liegt eine Aufgabe der vorliegenden Erfindung darin, ein relativ einfaches, wirtschaftlich durchführbares Verfahren zur Reinigung von α-, β-oder γ-substituierten Carbonsäuren aus wäßrigen Lösungen bereitzustellen.

Diese Aufgabe wird gelöst durch ein Verfahren zur Reinigung (im folgenden öfters "Reinigungsverfahren") einer α-, β- oder γ-substituierten Carbonsäure oder eines Salzes oder Esters davon oder eines Gemischs aus zwei oder mehr davon aus einer wäßrigen Lösung, die eine α-, β- oder γ-substituierte Carbonsäure oder ein Salz oder einen Ester davon und mindestens ein Polyol oder mindestens einen Aminoalkohol und ggf. mindestens ein Kation einer starken Base enthält, wobei diese Lösung mittels Elektrodialyse behandelt wird.

Das erfindungsgemäße Verfahren eignet sich insbesondere zur Reinigung einer α-, β- oder γ-substituierten Carbonsäure oder eines Salzes davon aus Destillationsrückständen, die bei der Racematspaltung eines Gemischs aus zwei Enantiomeren eines primären oder sekundären Amins anfallen.

Demgemäß kann das erfindungsgemäße Verfahren zur Rückgewinnung der oben definierten Carbonsäuren durch Behandlung mittels Elektrodialyse auch als eine Stufe in einem Verfahren zur Spaltung eines optisch aktiven Amids zu einer Carbonsäure oder einem Salz davon und einem optisch aktiven Amin unter Erhalt des Chiralitätszentrums, bzw. als eine Stufe in einem Verfahren zur Racematspaltung eines Gemischs aus zwei Enantiomeren eines primären oder sekundären Amins eingesetzt werden. Derartige Verfahren sind in der PCT/EP/96/03948 bzw. der WO 95/08636 beschrieben, deren Inhalt insbesondere bzgl. der allgemeinen Bedingungen zur Durchführung der Verfahren und der darin bevorzugt eingesetzten Verbindungen und Reagenzien voll umfänglich in die vorliegende Anmeldung durch Bezugnahme einbezogen wird.

Somit betrifft die vorliegende Erfindung auch ein Verfahren zur Spaltung eines optisch aktiven Amids zu einer Carbonsäure oder einem Salz davon und einem optisch aktiven Amin unter Erhalt des Chiralitätszentrums, das eine Hydrolyse des Amids in Gegenwart mindestens eines Polyols oder mindestens eines Aminoalkohols und mindestens eines Kations einer starken Base umfaßt, wobei die erhaltene α-, β- oder γ-substituierte Carbonsäure oder das Salz davon nach dem Reinigungsverfahren gemäß der vorliegenden Erfindung gereinigt wird, sowie ein Verfahren zur Racematspaltung eines Gemischs aus zwei Enantiomeren eines primären oder sekundären Amins, das folgende Schritte umfaßt:
(1) Umsetzung des Gemischs aus zwei Enantiomeren des Amins mit einem Ester, dessen Säurekomponente ein Halogen-, Stickstoff-, Sauerstoff-, Phosphor- oder Schwefelatom, gebunden an ein Kohlenstoffatom in α-, β- oder γ-Position zum Carbonylkohlenstoffatom, trägt, unter spezifischer Katalyse mit einer Hydrolase,
(2) Trennung des einen, enantioselektiv acylierten Amins (Amids) vom nicht umgesetzten, anderen Enantiomer des Amins,
(3) anschließende Hydrolyse des acylierten Amins (Amids) sowie Reinigung und Rückgewinnung einer entsprechenden α-, β- oder γ-substituierten Carbonsäure oder eines Salzes davon durch ein Reinigungsverfahren, wie oben definiert.

Zur weiteren Erläuterung des Anmeldungsgegenstands sind Figuren 1 bis 4 beigefügt:
- Fig. 1: zeigt schematisch das erfindungsgemäße Verfahren zur Racematspaltung ausgehend von einem racemischen primären aromatischen Amin;
- Fig. 2: ist eine Prinzipskizze der konventionellen Zwei-Kreis-Elektrodialyse am Beispiel eines Natriumcarboxylats;
- Fig. 3: ist eine Prinzipskizze der bipolaren Drei-Kreis-Elektrodialyse am Beispiel eines Natriumcarboxylats;
- Fig. 4: ist eine Prinzipskizze der bipolaren Zwei-Kreis-Elektrodialyse am Beispiel der Auftrennung eines Natriumcarboxylats und eines Alkanolamins.

Dabei bedeuten in den Zeichnungen:
(1) Diluatkreis
(2) Säurekreis
(3) Basekreis
(4) Konzentratkreis

- A: Anode
- K: Kathode
- AM: Anionenaustauschermembran
- KM: Kationenaustauschermembran
- BM: bipolare Membran

- E: Enzym
- ED: Elektrodialyse
- AA: R-Amin (Abtrennung)

Das erfindungsgemäße Verfahren zur Reinigung einer α-, β- oder γ-substituierten Carbonsäure oder eines Salzes oder Esters davon oder eines Gemischs aus zwei oder mehr davon (im folgenden öfters auch als "Carbonsäure" bezeichnet) aus einer wäßrigen Lösung, wie oben definiert, eignet sich prinzipiell für die Reinigung aller Carbonsäuren dieses Typs, wobei als Substituenten in α-, β- oder γ-Position zur Carboxylgruppe vorzugsweise elektronenreiche Heteroatome, wie z.B. ein Halogen-, Stickstoff-, Sauerstoff-, Phosphor- oder Schwefelatom, insbesondere Sauerstoff, vorhanden sind.

Das Heteroatom kann ggf. mit weiteren Gruppen, wie z.B. Alkylgruppen, verknüpft sein.

Besonders geeignete Carbonsäuren oder deren Salze oder deren Ester besitzen eine Struktur der allgemeinen Formel (I) 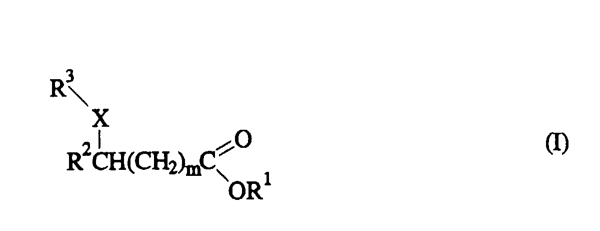 worin
- R¹: Wasserstoff, C₁- bis C₁₀-Alkyl oder ein Alkalimetallion, vorzugsweise Na⁺ oder K⁺, ist,
- R²: C₁- bis C₁₀-Alkyl oder Wasserstoff ist,
- R³: Wasserstoff, C₁- bis C₁₀-Alkyl oder ggf. durch NH₂, OH, C₁- bis C₄-Alkyl oder C₁- bis C₄-Alkoxy oder Halogen substituiertes Phenyl ist,
- X: ein Halogenatom, vorzugsweise Fluor (kein R³ vorhanden), Stickstoff, Sauerstoff, Phosphor oder Schwefel, insbesondere Sauerstoff, ist, und
- m: 0, 1 oder 2 ist.

Unter den Verbindungen, die unter die obige Strukturformel fallen, werden vorzugsweise C₁- bis C₄-Alkoxyderivate niederer Carbonsäuren, wie z.B. Methoxy- oder Ethoxyessigsäure, Methoxy- oder Ethoxypropionsäure und Methoxy- oder Ethoxybuttersäure, bzw. deren Salze, wie z.B. Natriummethoxyacetat, Natriummethoxypropionat oder Natriummethoxybutyrat weiter bevorzugt Methoxyessigsäure oder ein Salz davon, insbesondere deren Natriumsalz eingesetzt. Als Ester werden vorzugsweise die Methyl-, Ethyl-, Propyl- oder n-, sek.- oder tert.-Butylester der oben genannten Säuren eingesetzt.

Die zu reinigenden Lösungen weisen neben der oben definierten Carbonsäure bzw. deren Salz oder Ester in den zu reinigenden Lösungen als das mindestens eine Polyol oder den mindestens einen Aminoalkohol und ggf. als das mindestens eine Kation einer starken Base vorzugsweise Komponenten auf, die den in der PCT/EP/96/03948 detailliert beschriebenen Polyolen, Aminoalkoholen und Alkali- oder Erdalkalihydroxiden entsprechen. In der Regel sind jedoch in den zu reinigenden Lösungen, insbesondere dann, wenn es sich um Lösungen handelt, die direkt aus den Verfahren gemäß der WO 95/08636 und der PCT/EP/96/03948 beschriebenen Verfahren erhalten werden, als Polyol Ethylenglycol oder Diethylenglycol, als Aminoalkohol Ethanolamin oder Diethanolamin und als Kationen einer starken Base Natrium- und/oder Kaliumionen aus Natrium- und/oder Kaliumhydroxid vorhanden.

Der Begriff "Kationen einer starken Base" umfaßt alle Kationen, die sich von Basen ableiten, die in den hier in Rede stehenden Lösungen weitgehend dissoziert sind. Dabei sind vorzugsweise Kationen zu nennen, die sich von Basen mit einem pK_{B}-Wert von 0 ± 3,5 ableiten. Insbesondere zu nennen sind Alkali- und/oder Erdalkalikationen (aus Alkali- und/oder Erdalkalihydroxid) und NR'₄⁺-Kationen, wobei die Gruppen R' gleich oder verschieden sind, und jeweils Wasserstoff oder Alkyl, insbesondere Methyl, bedeuten. Nicht selten fallen jedoch die im erfindungsgemäßen Reinigungsverfahren zu behandelnden Lösungen, z.B. Destillationsrückstände aus den oben beschriebenen Verfahren zur Racematspaltung eines Gemischs aus zwei Enantionmeren eines primären oder sekundären Amins, bzw. zur Spaltung von optisch aktiven Amiden zu Carbonsäuren und optisch aktiven Aminen unter Erhalt des Chiralitätszentrums, in einer Form an, die nicht direkt der Elektrodialyse unterworfen werden kann.

Oftmals sind diese Lösungen hochviskos und enthalten Bestandteile, die die bei der Elektrodialyse verwendeten Membranen beschädigen können.

Demgemäß sind derartige Rohlösungen vor dem Einsatz in dem erfindungsgemäßen Verfahren noch einer Aufarbeitung, wie z.B. einer Verdünnung, einer Destillation, Filtration oder auch einem selektiven Ionenaustausch zu unterziehen.

Bei der Verwendung von Anionenaustauschermembranen (AM) in der Elektrodialyse ist in der Regel aufgrund der Alkali-Labilität dieser Membranen die Neutralisation des in den Lösungen ggf. vorhandenen überschüssigen NaOH und die Einstellung eines pH-Wertes von ungefähr 10 erforderlich. Bei Verwendung der seit einigen Jahren kommerziell erhältlichen alkalistabilen AM's, z.B. AMH (Tokuyama Corp.) und AMP (Asahi Glass), kann ggf. auf eine derartigen pH-Wert-Einstellung verzichtet werden. Vorzugsweise wird jedoch auch bei Verwendung dieser Membranen eine pH-Wert-Einstellung, wie oben definiert, vorgenommen. Zur Neutralisation können dabei alle membrangängigen und destillativ von den oben definierten Carbonsäuren abtrennbaren Säuren, wie z.B. Ameisensäure, verwendet werden.

In den im Rahmen des erfindungsgemäßen Reinigungsverfahrens eingesetzten wäßrigen Lösungen beträgt die Konzentration der Carbonsäure 5 bis 30, vorzugsweise 5 bis 25 und insbesondere 5 bis 15 Gew.-%. Der Gehalt an Polyol oder Aminoalkohol beträgt im allgemeinen 35 bis 85, vorzugsweise 45 bis 85 und insbesondere 15 bis 40 Gew.-%. Der Anteil an Wasser in der erfindungsgemäß eingesetzten Lösung beträgt im allgemeinen 40 bis 80 Gew.-%, der Anteil an Base, vorzugsweise Alkali- oder Erdalkalihydroxid (falls vorhanden) beträgt im allgemeinen 0,5 bis 5 Gew.-%.

Im Rahmen der vorliegenden Erfindung wird die Elektrodialyse als konventionelle Zwei-Kreis-Elektrodialyse, bipolare Zwei-Kreis-Elektrodialyse oder bipolare Drei-Kreis-Elektrodialyse oder als Kombination aus zwei oder mehr davon durchgeführt.

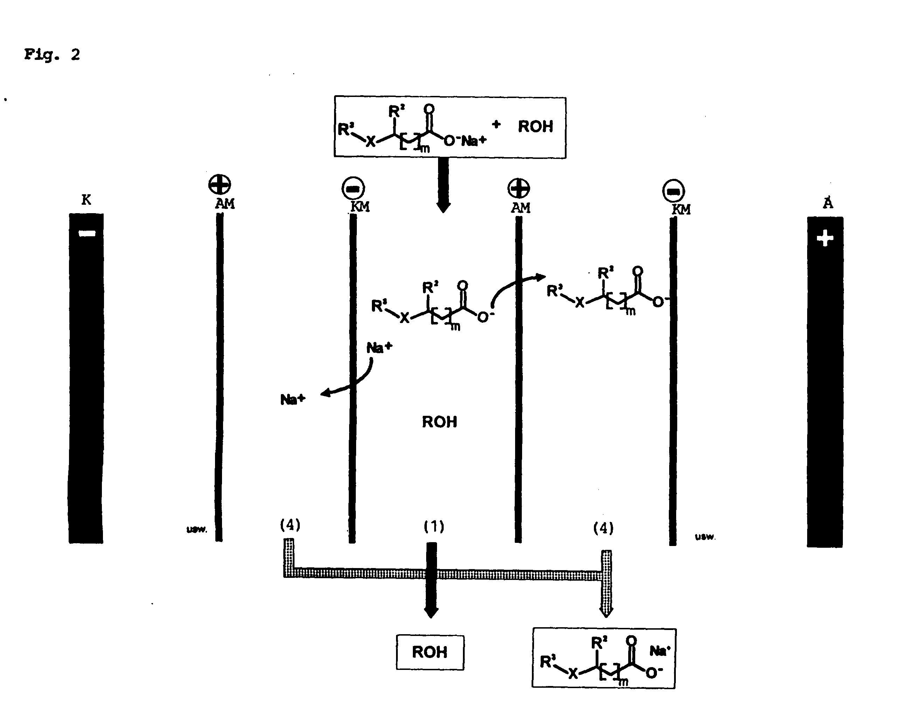

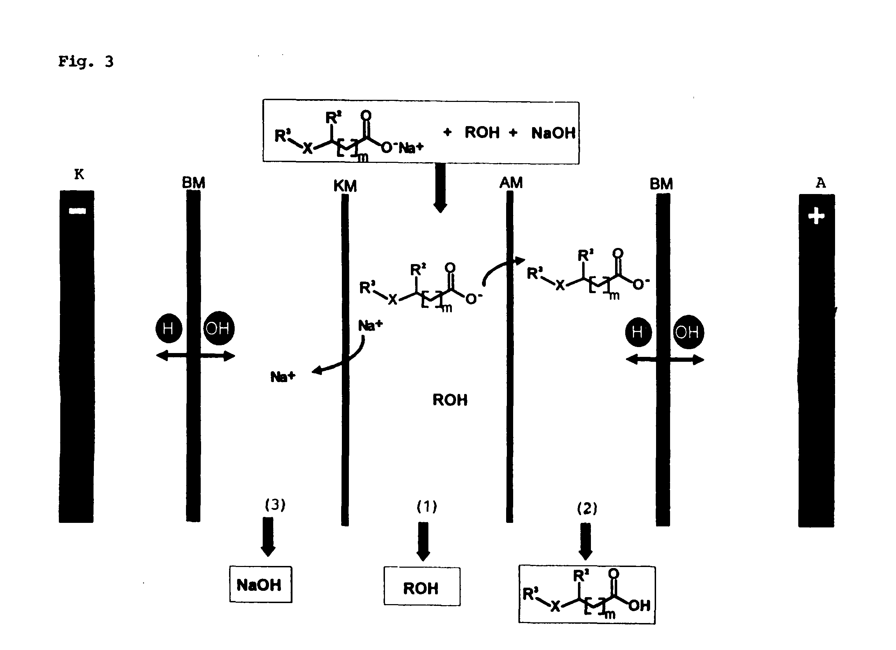

Sofern die aufzuarbeitende wäßrige Lösung neben der α-, β- oder γ-substituierten Carbonsäure oder einem Salz oder einem Ester davon mindestens ein Polyol und mindestens ein Kation einer starken Base enthält, wird diese vorzugsweise zunächst mittels konventioneller Zwei-Kreis-Elektrodialyse und anschließend mittels bipolarer Drei-Kreis-Elektrodialyse behandelt. Dabei findet im Rahmen der konventionellen Zwei-Kreis-Elektrodialyse eine Primärabreicherung der in der wäßrigen Lösung vorhandenen Carbonsäure oder des Salzes oder des Esters davon statt, wobei das Polyol weitgehend im Diluatkreis verbleibt und ein Konzentrat enthaltend die Carbonsäure bzw. deren Salz, erhalten wird. Das dabei erhaltene Konzentrat wird als Diluat in die 3-Kreis-Elektrodialyse eingebracht, wobei eine weitere Abtrennung des Polyols erreicht wird, und das Salz der Carbonsäure in Base und freie Carbonsäure gespalten wird. Eine schematische Darstellung der beiden Varianten wird in den später genauer erläuterten Figuren 2 und 3 gezeigt.

Sofern die aufzuarbeitende wäßrige Lösung neben der oben erwähnten Carbonsäure bzw. eines Salzes oder eines Esters davon einen Aminoalkohol und ein Kation einer starken Base enthält, wird diese vorzugsweise durch bipolare Zwei-Kreis-Elektrodialyse behandelt.

Die konventionelle Zwei-Kreis-Elektrodialyse ist an sich bekannt und wird u.a. in der EP-B-0 381 134 beschrieben, deren Inhalt bezüglich der konventionellen Zwei-Kreis-Elektrodialyse vollumfänglich in die vorliegende Anmeldung miteinbezogen wird.

Eine Prinzipskizze dieser Elektrodialyse zeigt Figur 2.

Bei dieser Elektrodialyse-Variante wird eine Apparatur eingesetzt, die eine positive (Anode (A)) und eine negative (Kathode (K)) als großflächige Elektroden aufweist. Der Raum zwischen diesen Elektroden wird durch eine Vielzahl von abwechselnd angeordneten Kationen (KM)- und Anionen (AM)-Austauschermembranen in eine Vielzahl schmaler, durch die Membranen voneinander getrennter Kammern, die man auch als Diluatkreis (1) und Konzentratkreis (4) bezeichnet, aufgeteilt. Dabei stellen Kammern, die kathodenseitig eine Anionenaustauschermembran und anodenseitig eine Kationenaustauschermembran haben, die sog. Konzentratkammern oder Konzentratkreise dar, während Kammern, die anodenseitig die Anionenaustauschermembran und kathodenseitig die Kationenaustauschermembran haben, die Diluatkammern bzw. den Diluatkreis bilden.

Zur Durchführung des erfindungsgemäßen Verfahrens werden die Diluatkreise mit der aufzureinigenden wäßrigen Lösung beispielsweise enthaltend ein Na-Salz einer Carbonsäure in der m, X, R² und R³ wie bzgl. der Struktur (I) definiert sind, und einen Aminoalkohol oder ein Polyol dargestellt durch ROH, die Konzentratkreise mit einem wäßrigen Elektrolyt befüllt. Die Kammern, in denen sich die Elektroden befinden und ggf. noch die unmittelbar an diese angrenzenden Nachbarkammern werden mit einer Elektrodenspüllösung, in der Regel einer Natriumsulfatlösung, beaufschlagt.

Unter dem Einfluß der an die Elektroden angelegten Spannung wandern die Ionen durch die für sie durchlässige Membran aus dem Diluatkreis hinaus in den Konzentrationskreis. Durch die folgende, für die entsprechende Ionensorte undurchlässige Membran ist eine Weiterwanderung nicht möglich und das Ion verbleibt im Konzentratkreis. Die Flüssigkeiten in den Diluat-, Konzentrat- und Elektrodenkreisen werden in getrennten Kreisläufen umgepumpt, ggf. unter Einschaltung von Reservoiren.

In Abänderung zur aus der EP-B 0 381 134 bekannten konventionellen Zwei-Kreis-Elektrodialyse kann auch eine Membrananordnung unter Verwendung von bipolaren Membranen eingesetzt werden. Bipolare Membranen stellen Laminate aus Anionen- und Kationenaustauschermembranen dar. Sie zeichnen sich gegenüber monopolaren Anionen- bzw. Kationenaustauschermembranen dadurch aus, im elektrischen Feld der Elektrodialyse eine effiziente Wasserspaltung zu katalysieren und dienen somit zur gleichzeitigen Bereitstellung von H⁺- und OH⁻-Äquivalenten.

Die Eigenschaften der bipolaren Membranen können in der in Figur 3 dargestellten Weise zum Erhalt der reinen Säure verwendet werden. Die Funktionsweise wird wiederum anhand der Behandlung einer wäßrigen Lösung eines Na-Salzes einer Carbonsäure der Struktur (I) und eines Polyols oder Aminoalkohols ROH, wie jeweils bzgl. Figur 2 erläutert, im folgenden kurz beschrieben.

Verwendung findet eine Drei-Kreis(Kammer)-Anordnung (bipolare Drei-Kreis-Elektrodialyse), bestehend aus Diluat (1)-, Säure (2)- und Base (3)-Kreis. Die Drei-Kreis-Anordnung wird durch alternierende Aufeinanderfolge der jeweiligen Austauschermembran erzielt:
... BM BK KM DK AM SK BM...
KM = Kationenaustauschermembran; BM = bipolare Membran;
AM = Anionenaustauschermembran;
BK = Basekreis; SK = Säurekreis; DK = Diluatkreis

Dem Diluatkreis wird die aufzutrennende wäßrige Lösung, die Carbonsäure bzw. deren Salz oder Ester und das Lösungsmittel enthält, zugeführt. Im Säurekreis wird eine verdünnte Carbonsäurelösung (z.B. 0,5%-ig) vorgelegt, während im Basekreis eine entsprechend verdünnte Base, z.B. NaOH vorgelegt wird. Beim Einschalten des Elektrodialysestroms erfolgt analog der konventionellen Elektrodialyse eine Wanderung der Kationen (Beispiel Na⁺) aus dem Diluatkreis in den Basekreis, während das Säureanion in den Säurekreis wandert. Im Base- bzw. Säurekreis wird mit den OH⁻- bzw. H⁺-Ionen aus der simultanen Wasserspaltung der bipolaren Membran die Base (NaOH) bzw. die α-, β- oder γ-substituierte Carbonsäure generiert.

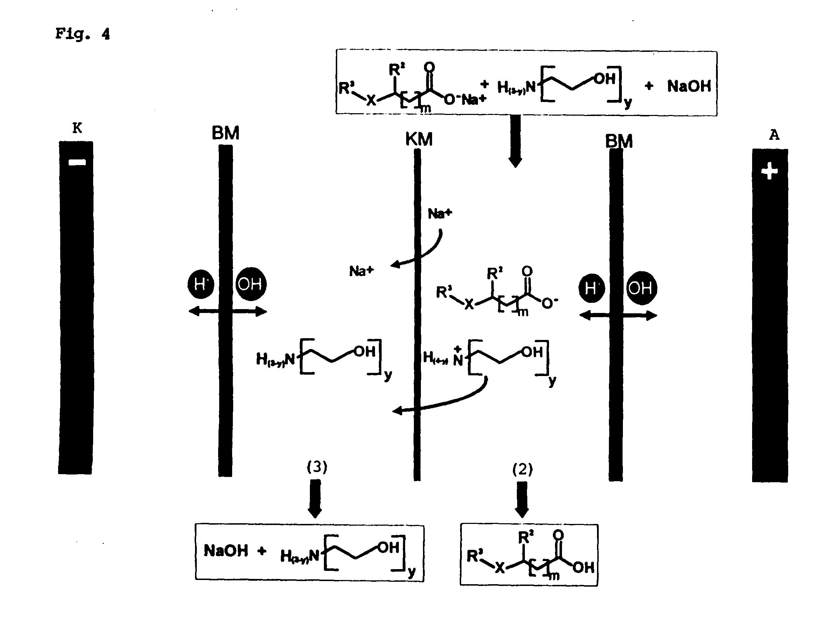

Sofern in der Lösung ein Alkanolamin vorhanden ist, wird vorzugsweise neben der bipolaren Drei-Kreis-Elektrodialyse auch eine bipolare Zwei-Kreis-Elektrodialyse eingesetzt. Eine derartige Anordnung ist in Fig. 4 gezeigt und besteht aus Base(3)- und Säure(2)-Kreis. Diese Anordnung wird durch alternierende Aufeinanderfolge der jeweiligen Ionenaustauschermembranen erzielt:
... BM BK KM SK BM ...
BM = bipolare Membran; KM = Kationenaustauschermembran;
BK = Basekreis; SK = Säurekreis

Bei dieser Anordnung wird die erfindungsgemäß zu behandelnde Lösung im Säurekreis eingesetzt und im Basekreis eine stark verdünnte Lösung einer Base (z.B. NaOH, 0,5 %ig) vorgelegt. Bei Einschalten des Elektrodialysestroms erfolgt ebenfalls eine Wanderung der positiv geladenen Ionen durch die Kationenaustauschermembran hindurch aus dem Säurekreis in den Basekreis, wobei dort mit den aus der Wasserspaltung aus der bipolaren Membran austretenden OH⁻-Ionen die entsprechende Basen gebildet wird. Die Anionen verbleiben im Säurekreis und bilden mit den dort vorhandenen H⁺-Ionen die entsprechende freie Säure. Weitere Einzelheiten lassen sich z.B. bei K.N. Mani, J. Membr. Sci. 58 (1991), S. 117-38 entnehmen.

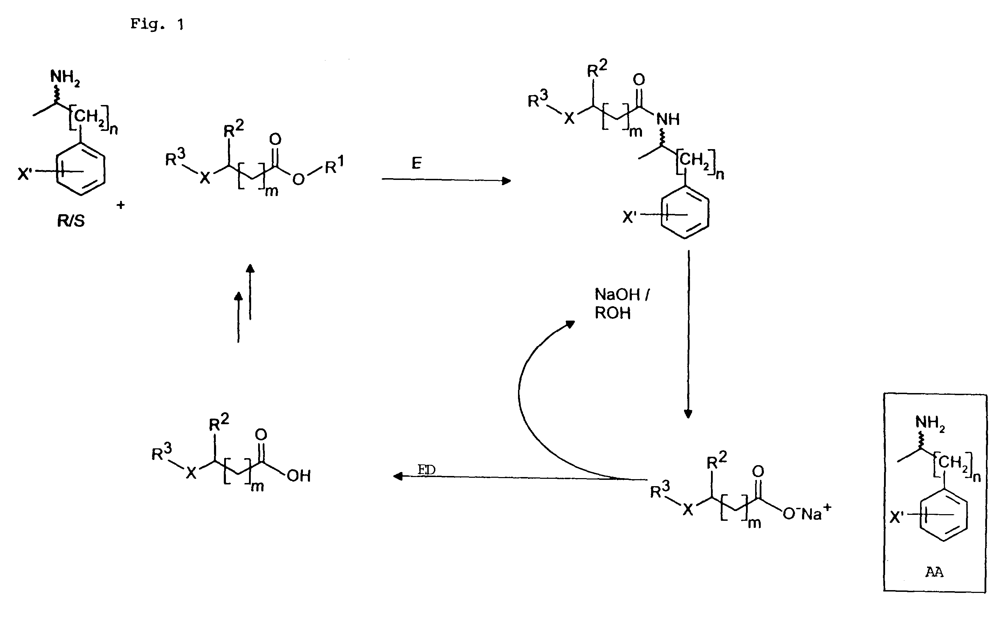

Die Nomenklatur der eingesetzten Verbindungen entspricht dabei dem zu Fig. 1 und 2 Gesagten, wobei y - als zusätzliche Variable - die Werte 0, 1, 2, 3, 4 annehmen kann.

Das erfindungsgemäße Verfahren wird vorzugsweise bei 10 bis 50 °C, insbesondere zwischen 20 und 30 °C durchgeführt. Die Stromdichte bei der konventionellen Zwei-Kreis-Elektrodialyse variiert dabei zwischen 100 und 700 A/m², vorzugsweise zwischen 50 und 500 A/m². Bei der bipolaren Drei-Kreis-Elektrodialyse variiert die Stromdichte zwischen 1 und 2.000 A/m², vorzugsweise zwischen 500 und 1.500 A/m². Diese Werte treffen auch für die bipolare Zwei-Kreis-Elektrodialyse zu.

Bei der im Rahmen des vorliegenden Verfahrens durchgeführten Elektrodialyse werden handelsübliche Ionenaustauschermembranen eingesetzt.

Diese bestehen vorzugsweise aus organischen Polymeren, die ionenaktive Seitenketten aufweisen. Kationenaustauschermembranen enthalten Sulfonat-oder Carboxylgruppen in der Polymermatrix, Anionenaustauschermembranen weisen tertiäre oder quartäre Aminogruppen und Substituenten des polymeren Grundmaterials auf. Besonders geeignet als polymeres Grundmaterial für die Ionenaustauschermembranen sind Copolymerisate von Styrol und Divinylbenzol. Als verwendbare Anionenaustauschermembranen sind beispielsweise zu nennen: Tokuyama AMI, AM2, AM3, AMX, AMH, AFN, Asahi Glass AMP, AMV. Als Kationenaustauschermembranen sind Tokuyama CM1, CM2, CMX, CMH und Asahi Glass CMV beispielhaft zu nennen. Als Beispiele für bipolare Membranen sind Tokuyama BP1 und Aqualytics Membranen zu nennen.

Aufgrund der oben bereits beschriebenen Alkali-Labilität von Anionenaustauschermembranen sind im Rahmen des vorliegenden Verfahrens die Ausführungsformen, in denen auf die Verwendung von Anionenaustauschermembranen verzichtet werden kann, bevorzugt.

In der Regel ist es im Rahmen des erfindungsgemäßen Verfahrens möglich, durch einmalige Elektrodialyse eine Lösungsmittel-Abreicherung (Polyol-/Aminoalkohol-Abreicherung) von ungefähr 80%, vorzugsweise bis zu ungefähr 90%, jeweils bezogen auf den Ausgangsgehalt, zu erreichen. Dies wird in der Regel bei Verwendung von relativ verdünnten Ausgangslösungen, die die Carbonsäure in einer Konzentration von 5 bis 15 Gew.-% enthalten, erreicht.

Alternativ dazu werden im Rahmen des erfindungsgemäßen Verfahrens die Lösungen einer doppelten Elektrodialyse unterzogen, bei der auch konzentriertere Ausgangslösungen, die, wie oben bereits erwähnt, einen Gehalt an Carbonsäure oder eines Salzes davon von bis zu 30 Gew.-% aufweisen können, verarbeitet werden. Mittels dieser doppelten Elektrodialyse ist eine Abreicherung des Lösungsmittels bis zu über 95% möglich.

Da, wie oben erwähnt, nach einfacher Elektrodialyse im günstigsten Fall im Konzentratkreis/Säurekreis ein Stoffmengenverhältnis von Carbonsäure zu Lösungsmittel von ungefähr 15:1 erreicht werden kann und selbst nach doppelter Elektrodialyse noch mit Lösungsmittelspuren im Konzentrat zu rechnen ist, muß davon ausgegangen werden, daß bei der sich in der Regel an das erfindungsgemäße Verfahren anschließenden Destillation zur Abtrennung der Carbonsäure noch Ausbeuteverluste durch die Bildung von schwerlöslichen Reaktionsprodukten zwischen Lösungsmittel und Carbonsäure, wie z.B. schwerflüchtige Mono-, Di oder Triester auftreten, was zu Ausbeuteverlusten bezüglich der Carbonsäure führt.

Demgemäß wird in einer weiteren Ausführungsform der vorliegenden Erfindung der Sumpf der bei der nach der Elektrodialyse im erfindungsgemäßen Reinigungsverfahren durchgeführten Destillation mit einer stöchiometrischen Menge an verdünnter Base (beispielsweise 3,5%ige) Natronlauge versetzt, was in der Regel zur vollständigen Esterspaltung unter Bildung eines Salzes der gewünschten Carbonsäure und des Polyols oder Aminoalkohols, also z.B. zur Bildung von Natriummethoxyacetat und Ethylenglycol, führt. Das so erhaltene Gemisch kann dann zusammen mit frischer Lösung nochmals einer Elektrodialyse zugeführt werden.

Diese Variante des erfindungsgemäßen Verfahrens stellt eine Alternative zur zweimaligen Behandlung der zu reinigenden Lösungen mittels Elektrodialyse dar.

Jedenfalls ermöglicht es diese Alternative, einen signifikanten Restgehalt an Polyol oder Aminoalkohol vor der abschließenden Destillation zu akzeptieren.

In einer weiteren Ausführungsform der vorliegenden Erfindung wird die wäßrige Lösung während der Elektrodialyse oder nach dem Durchlaufen der Elektrodialyse über ein Kationenaustauschermodul geleitet, um eine weitere Abreicherung des Lösungsmittels, bzw. eine weitere Reinigung der Carbonsäure zu erreichen.

Der Zeitpunkt der Kopplung von Elektrodialyse mit einem Kationenaustauscherprozeß wird so gewählt, daß die vornehmliche Abreicherung von Kationen über die Elektrodialyse bereits erfolgt ist.

Das "Einschalten" des Kationenaustauscherprozesses geschieht in der Regel beim Erreichen einer definierten Leitfähigkeit im Diluat der Elektrodialyse, die mit einer Abreicherung an Base von 80 bis 99%, vorzugsweise 90 bis 99%, korreliert, d.h. die Elektrodialyse wird vorzugsweise beim Erreichen einer Leitfähigkeit von 20 mS/cm oder weniger, weiter bevorzugt bei einer Leitfähigkeit von 10 mS/cm oder weniger und insbesondere bei einer Leitfähigkeit von ungefähr 5 mS/cm oder weniger mit einem Kationenaustauscherprozeß gekoppelt.

Somit wird bei dieser Variante des erfindungsgemäßen Verfahrens zunächst die Elektrodialyse vorzugsweise bis zu einer Leitfähigkeit des Diluats von 20 mS/cm oder weniger betrieben und anschließend das in der Elektrodialyse erhaltene Diluat über ein Kationenaustauschermodul geleitet.

Darüber hinaus kann das erfindungsgemäße Verfahren auch so durchgeführt werden, daß zunächst die Elektrodialyse bis zu einer Leitfähigkeit des Diluats von 20 mS/cm oder weniger alleine betrieben wird und anschließend das Diluat sowohl der Elektrodialyse unterworfen wird als auch über ein Kationenaustauschermodul geleitet wird.

Als Kationenaustauschermodule im Kationenaustauscherprozeß können Vorrichtungen, wie z.B. eine Säule, die mit den oben bezüglich der Elektrodialyse beschriebenen Kationenaustauschern in Form von Pulver, Perlen, Granulaten, usw. befüllt sind, eingesetzt werden. Es kommen prinzipiell alle Kationenaustauscher auf Polymerbasis, d.h. sowohl schwach- als auch starksaure Kationenaustauscher in Frage. Beispielhaft zu nennen sind Dowex 50 W-Typen, Amberlite IR 120 bzw. IR 400, Lewatit S 100 und Duolite C 26.

In einer weiteren Ausführungsform des erfindungsgemäßen Verfahrens wird eine α-, β- oder γ-substituierte Carbonsäure oder ein Salz davon in Form einer wäßrigen Lösung, die bei der Racematspaltung eines optisch aktiven Amids zu einer Carbonsäure und einem optisch aktiven Amin unter Erhalt des Chiralitätszentrums erhalten wurde, erfindungsgemäß gereinigt.

Somit kann das erfindungsgemäße Verfahren auch als Bestandteil des Verfahrens zur Spaltung von optisch aktiven Amiden zu Carbonsäuren und optisch aktiven Aminen unter Erhalt des Chiralitätszentrums, wie in der PCT/EP/96/03948 beschrieben, bzw. als Bestandteil zu dem in der WO 95/08636 beschriebenen Verfahren zur Racematspaltung eines Gemischs aus zwei Enantiomeren eines primären oder sekundären Amins verwendet werden.

Fig. 1 gibt einen beispielhaften Überblick über das Gesamtverfahren am Beispiel eines aromatischen Amins der folgenden Struktur: 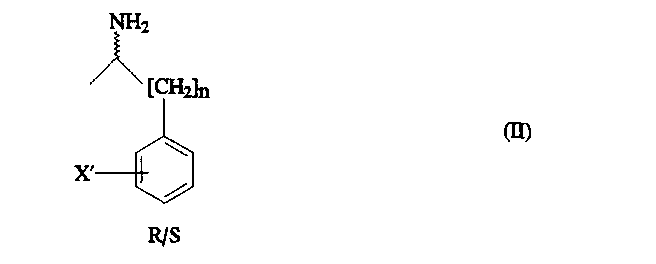 wobei X' übliche Substituenten für Aromaten, insbesondere Halogen, lineare und verzweigte Alkylgruppen mit 1 - 6 C-Atomen, lineare Alkoxygruppen mit 1 - 6 C-Atomen, Acylgruppen mit 1 - 6 C-Atomen, und Nitrilgruppen darstellt, und n die Werte 1, 2, 3, 4, 5, 6, usw. annimmt.

Im Rahmen dieses Gesamtverfahrens wird zunächst das Gemisch aus zwei Enantiomeren eines primären oder sekundären Amins mit einem Ester, dessen Säurekomponente ein Halogen-, Stickstoff-, Sauerstoff-, Phosphor-oder Schwefelatom, gebunden an ein Kohlenstoffatom in α-, β- oder γ-Position zum Carbonylkohlenstoff, trägt, unter spezifischer Katalyse mit einer Hydrolase in die beiden enantiomeren Formen überführt.

Als Ester eignen sich die bereits weiter oben detailliert beschriebenen Ester, die unter die Formel (I) fallen.

Die verwendbaren Hydrolasen sind ebenfalls detailliert in der WO 95/08636 beschrieben und werden hierin lediglich durch Bezugnahme auf dieses Dokument einbezogen.

In einem weiteren Schritt wird das eine, enantioselektiv acylierte Amin (Amid) vom nicht umgesetzten anderen Enantiomer des Amins getrennt, beispielsweise durch fraktionierte Destillation.

Anschließend wird das erhaltene optisch aktive Amid zu einer Carbonsäure und einem optisch aktiven Amin unter Erhalt des Chiralitätszentrums gespalten, wobei die Hydrolyse in Gegenwart mindestens eines Polyols oder mindestens eines Aminoalkohols und mindestens eines Kations einer starken Base durchgeführt wird.

Als Polyole bzw. Aminoalkohole kommen dabei die ebenfalls eingangs erwähnten bzw. in der WO 95/08636 beschriebenen Polyole und Aminoalkohole in Betracht.

Das während der Hydrolyse freigesetzte Amin wird destillativ abgetrennt und die so erhaltene, eine in α-, β- oder γ-Position substituierte Carbonsäure, mindestens ein Polyol oder mindestens einen Aminoalkohol und mindestens ein enthaltende Lösung dem erfindungsgemäßen Reinigungsverfahren unterzogen.

Die beim erfindungsgemäßen Reinigungsverfahren abschließend erhaltene Carbonsäure oder das Salz davon kann dann wiederum verestert werden und der Umsetzung der racemischen Amine als Veresterungsagens zugegeben werden.

### BEISPIELE

### 1. Konventionelle Elektrodialyse

### Allgemeine Bedingungen

Die konventionelle Elektrodialyse wurde in einem 2-Kreis-Elektrodialysemodul mit einer alternierenden Anordnung von Kationen- und Anionenaustauschermembranen (s. Fig. 2) durchgeführt, wobei als Anionenaustauschermembranen AM3 und als Kationenaustauschermembranen CM2, jeweils Tokuyama, Verwendung fanden. Die Membranen waren im Abstand von 0,5mm zueinander angeordnet.

Das Modul war aus je 5 Diluat- und Konzentratkammern aufgebaut, was einer effektiven Gesamtmembranfläche von 3,78 dm² entsprach. Als Anoden-und Kathodenmaterial wurden platinierte Titan-Elektroden eingesetzt. Die Nennstromdichte betrug 5,3 A/dm², der maximale Spannungsabfall pro Zelle (1 Paarung aus Diluat- und Konzentratkammer) wurde auf 2V begrenzt. Die Elektrodialysetemperatur betrug 40°C.

### Beispiel 1

Im Diluatkreis wurden 800g einer wäßrigen Lösung, bestehend aus Natriummethoxyacetat (NaMes) (20,9%) und Ethylenglykol (EG) (34,9%) eingesetzt, was einem NaMes/EG Molverhältnis von y ≈ 0,3 entsprach.

Im Konzentratkreis wurden 500g einer 2%-igen, wäßrigen Natriummethoxyacetat-Lösung vorgelegt. Der Elektrolytkreis wurde mit einer 5%-igen, wäßrigen Natriumsulfatlösung befüllt.

Nach 5h wurde die Übertragung von Natrium- und Methoxyacetat-Ionen in den Konzentratkreis durch Abschalten des Gleichrichters beendet. 778 g Konzentrataustrag folgender Zusammensetzung wurden erhalten:
NaMes 18,23%; EG 2,78%; y≈3,6.

### Beispiel 2

In Abänderung zu Beispiel 1 wurden 1200g einer wäßrigen Diluatlösung der folgenden Zusammensetzung eingesetzt:
NaMes 14,0%; EG 23,5%; y≈0,3.

Nach 5h Elektrodialyse wurden 845g Konzentrat der folgenden Zusammensetzung erhalten:
NaMes 17,1%; EG 1,4%; y≈6,8.

### Beispiel 3

In Abänderung zu Beispiel 1 wurden 2400g einer wäßrigen Diluatlösung der folgenden Zusammensetzung eingesetzt:
NaMes 7,0%; EG 11,63; y≈0,3

Nach 5h Elektrodialyse wurden 917g Konzentrat der folgenden Zusammensetzung erhalten:
NaMes 15,7%; EG 0,6%;y≈14,4.

### Beispiel 4

845g Konzentrataustrag (aus Beispiel 2; Zusammensetzung s.o.) wurden erneut als Diluateinsatz der Elektrodialyse unterworfen.

Nach 5h Elektrodialyse wurden 752g Konzentrat der folgenden Zusammensetzung erhalten:
NaMes 16,2% EG < 0,05%; y≈18,0.

### 2. Bipolare Elektrodialyse (3-Kreis)

### Allgemeine Bedingungen

Die bipolare 3-Kreis-Elektrodialyse wurde mit einer alternierenden Anordnung folgender Membranen durchgeführt:

Bipolare Membran (Aqualytics, USA) - Anionenaustauschermembran (AM3, Tokuyama Corp., JP) - Kationenaustauschermembran (CM2, Tokuyama Corp., JP). Die Membranen wurden im Abstand von 1,0 mm zueinander angeordnet.

Das Modul war aus je 5 Diluat-, Säure- und Basekammern aufgebaut, was einer aktiven Gesamtmembranfläche von 27 dm² entsprach. Als Anodenmaterial wurde Nickel, als Kathodenmaterial wurde Edelstahl verwendet. Die Nennstromdichte betrug 8,0 A/dm², der maximale Spannungsabfall pro Zelle (Segment aus Säure-, Diluat-, und Basekreis) wurde auf 4,0 V begrenzt. Die Elektrodialysetemperatur betrug 40°C.

### Beispiel 5

### Startspezifikation der eingesetzten Lösungen:

a) 2400g Diluatkreis der folgenden Zusammensetzung:
   NaMes 14,0%; EG 23,3%; y≈0,3.
b) 800g Säurekreis der folgenden Zusammensetzung:
   Methoxyessigsäure/HMes, wäßrig 2,0%.
c) 1000g Basekreis der folgenden Zusammensetzung:
   Natriumhydroxid, wäßrig 2,0%.
d) 1000g Elektrolytkreis der folgenden Zusammensetzung:
   Natriumhydroxid, wäßrig 6,0%.

Nach 70 Minuten wurde die Übertragung von Methoxyacetat- bzw. Natrium-Ionen aus dem Diluat in den Säure- bzw. Basekreis durch Abschalten des Gleichrichters beendet. Es wurden folgende Elektrodialyseausträge erhalten:
a) 1808g Diluatkreis der folgenden Zusammensetzung:
   NaMes 1,9%; EG 25,4%; y≈0,04.
b) 1141g Säurekreis der folgenden Zusammensetzung:
   HMes, 21,5%; EG 1,7%; y≈7,0.
c) 1242g Basekreis der folgenden Zusammensetzung:
   Natriumhydroxid, 8,7%.

Die im Säurekreis erhaltene Methoxyessigsäure (HMes) wurde nach Abziehen des überschüssigen Wassers durch einfache Vakuum-Destillation isoliert.

### 3. Bipolare Elektrodialyse (2-Kreis)

### Allgemeine Bedingungen

Die bipolare 2-Kreis-Elektrodialyse wurde mit einer alternierenden Anordnung folgender Membranen durchgeführt:

Bipolare Membran (Aqualytics USA), Kationenaustauschermembran (CMX, Tokuyama Corp. JP). Die Membranen wurden im Abstand von 1,0mm zueinander angeordnet.

Das Modul war aus je 5 Säure- und Basekammern aufgebaut, was einer aktiven Gesamtmembranfläche von 9,29 dm² entsprach. Als Anodenmaterial wurde Nickel, als Kathodenmaterial wurde Edelstahl verwendet. Die Nennstromdichte betrug 8,0 A/dm², der maximale Spannungsabfall pro Zelle (Segment aus Säure- und Basekreis) wurde auf 3,5 V begrenzt. Die Elektrodialysetemperatur betrug 40 °C.

### Beispiel 6

### Startspezifikation der eingesetzten Lösungen

a) 3000g Säurekreis der folgenden Zusammensetzung:
   Triethanolamin/TEA 27,3%; NaMes 17,0% (NaMes/TEA Molverhältnis von y≈0,83); NaOH 1,6%;
b) 2500g Basekreis der folgenden Zusammensetzung:
   Natriumhydroxid, wäßrig 2,0%.
c) 1000g Elektrolytkreis der folgenden Zusammensetzung:
   Natriumhydroxid, wäßrig 6,0%.

Die Übertragung von Triethanolammonium- und Natrium-Ionen vom Säurekreis in den Basekreis wurde nach 590 min. bei Erreichen eines Säurekreis-pH's von 1,7 durch Abschalten des Gleichrichters beendet. Es wurde folgende Elektrodialyseausträge erhalten:
a) 822g Säurekreis der folgenden Zusammensetzung:
   TEA 1,1%; HMes 30,7%; y≈46.
b) 4650g Basekreis der folgenden Zusammensetzung:
   TEA 15,0%; NaMes 2,9%; NaOH 3,3%.

Der Säurekreisaustrag wurde am Rotationsverdampfer durch Abziehen von 546g Wasser aufkonzentriert (60°C Badtem.; 30mbar). Der Rückstand von 276g wurde einer einfachen Vakuumdestillation (70-130°C Sumpftemp.; 0,3 →0,01 mbar) unterzogen. Es wurden vier HMes-Fraktionen entsprechend einer HMes Gesamtmenge von 227,9g = 90,3% der Theorie erhalten. Die Fraktionen 3 und 4 (214,3g HMes = 85% der Theorie) wurden mit einer Reinheit von ≥99,0% zur enzymatischen Racematspaltung zurückgeführt. Die Fraktionen 1 und 2 gelangten in den Destillationsansatz eines Folgeversuches.

Der Basekreisaustrag diente einer erneuten Amidspaltung.

Zur Entsorgung fiel ein organisch/salzhaltiger Destillationsrückstand von 24g an.

### Beispiel 7

a) 3000g Säurekreis der folgenden Zusammensetzung:
   Triethanolamin/TEA 19,3%; NaMes 12,3% (NaMes/TEA Molverhältnis von y≈0,85); NaOH 1,0%.
b) 2500g Basekreis der folgenden Zusammensetzung:
   Natriumhydroxid, wäßrig 2,0%.
c) 1000g Elektrolytkreis der folgenden Zusammensetzung:
   Natriumhydroxid, wäßrig 6,0%.

Die Übertragung von Triethanolammonium- und Natrium-Ionen vom Säurekreis in den Basekreis wurden nach 426 min. bei Erreichen eines Säurekreis-pH's von 2,4 durch Abschalten des Gleichrichters beendet. Es wurden folgende Elektrodialyseausträge erhalten:
a) 1500g Säurekreis der folgenden Zusammensetzung:
   TEA 2,15%; Gesamt-Mes 17,0% (ber. HMes, verteilt auf freigestzte Carbonsäure = 15,7% und Methoxyacetat-TEA bzw.-Na-Salz 1,3%); y≈12.
b) 4050g Basekreis der folgenden Zusammensetzung:
   TEA 13,4%; NaMes 1,1%; NaOH 2,8%.

Der Säurekreisaustrag wurde am Rotationsverdampfer durch Abziehen von 1011g Wasser aufkonzentriert (60°C Badtemp.; 30mbar). Der Rückstand von 489g wurde einer einfachen Vakuumdestillation (70-140°C Sumpftemp.; 0,3→0,01mbar) unterzogen. Es wurden vier HMes Fraktionen entsprechend einer HMes Gesamtmenge von 199,7g erhalten. In diesen befanden sich 84,8% der im Säurekreisaustrag enthaltenen HMes bzw. 78,3% des im Säurekreisaustrag vorliegenden Methoxyacetats. Die Fraktionen 3 und 4 (178,7g HMes = 75,9/70,1% der Theorie) wurden vereinigt und enthielten HMes mit einer Reinheit von ≥95,8. Die Fraktionen 1 und 2 wurden in die Destillation eines Folgeversuches zurückgeführt.

Der Basekreisaustrag diente einer erneuten Amidspaltung.

Zur Entsorgung fiel ein organisch/salzhaltiger Destillationsrückstand von 72g an.

### Beispiel 8

Die Durchführung der Elektrodialyse entsprach den Angaben in Beispiel 7.

In Abänderung zu Beispiel 7 wurden 1500g analog zusammengesetzter Säurekreisaustrag über eine mit einem stark sauren Kationenaustauscher (Amberlite IR120/H⁺, ≈250 ml; ⌀ 40mm, Betthöhe 200mm) gepackte Säule vollentsalzt. Das gesammelte Ionenaustauscher-Eluat (≈ 2250g) wurde am Rotationsverdampfer durch Abziehen von Wasser aufkonzentriert (60°C Badtemp.; 30mbar). Der Rückstand von 257g wurde einer einfachen Vakuumdestillation (70-130°C Sumpftemp.; 0,3→0,01mbar) unterzogen. Es wurden zwei HMes Fraktionen entsprechend einer HMes-Gesamtmenge von 246,1g erhalten. Diese enthielten 96,5 % der im Säurekreisaustrag in Form von Salz/Säure enthaltenen Methoxyessigsäure. Die Fraktionen 1 und 2 wurden vereinigt und enthielten HMes mit einer Reinheit von ≥99,0%. Sie wurden in die enzymatische Racematspaltung zurückgeführt.

Der Basekreisaustrag diente einer erneuten Amidspaltung.

Zur Entsorgung fiel ein organisch/salzhaltiger Destillationsrückstand von 1,5g an.

## Patentansprüche

1. Verfahren zur Reinigung einer α-, β- oder γ-substituierten Carbonsäure oder eines Salzes oder eines Esters davon oder eines Gemischs aus zwei oder mehr davon aus einer wäßrigen Lösung, die eine α-, β- oder γ-substituierte Carbonsäure oder ein Salz oder einen Ester davon oder ein Gemisch aus zwei oder mehr davon und mindestens ein Polyol oder mindestens einen Aminoalkohol enthält, wobei diese Lösung mittels Elektrodialyse behandelt wird.

2. Verfahren nach Anspruch 1, wobei die wäßrige Lösung zusätzlich mindestens ein Kation einer starken Base enthält.

3. Verfahren nach Anspruch 1 oder 2, wobei die Elektrodialyse als konventionelle Zwei-Kreis-Elektrodialyse, bipolare Zwei-Kreis-Elektrodialyse oder bipolare Drei-Kreis-Elektrodialyse oder als Kombination aus zwei oder mehr davon durchgeführt wird.

4. Verfahren nach Anspruch 2 oder 3, wobei die wäßrige Lösung mindestens ein Polyol und mindestens ein Kation einer starken Base enthält und zunächst mittels konventioneller Zwei-Kreis-Elektrodialyse und anschließend mittels bipolarer Drei-Kreis-Elektrodialyse behandelt wird.

5. Verfahren nach Anspruch 2 oder 3, wobei die wäßrige Lösung mindestens einen Aminoalkohol und mindestens ein Kation einer starken Base enthält und durch bipolare Zwei-Kreis-Elektrodialyse behandelt wird.

6. Verfahren nach einem der vorstehenden Ansprüche, wobei die wäßrige Lösung während der Elektrodialyse oder nach dem Durchlaufen der Elektrodialyse über ein Kationenaustauschmodul geleitet wird.

7. Verfahren nach einem der vorstehenden Ansprüche, wobei die α-, β-oder γ-substituierte Carbonsäure oder ein Salz davon in Form einer wäßrigen Lösung, die bei der Racematspaltung eines optisch aktiven Amids zu einer Carbonsäure und einem optisch aktiven Amin unter Erhalt des Chiralitätszentrums erhalten wurde, vorliegt.

8. Verfahren nach einem der vorstehenden Ansprüche, wobei die α, β-oder γ-substituierte Carbonsäure oder das Salz davon, Methoxyessigsäure oder ein Salz davon ist.

9. Verfahren zur Spaltung eines optisch aktiven Amids zu einer Carbonsäure oder einem Salz davon und einem optisch aktiven Amin unter Erhalt des Chiralitätszentrums, das eine Hydrolyse des Amids in Gegenwart mindestens eines Polyols oder mindestens eines Aminoalkohols und mindestens eines Kations einer starken Base umfaßt, wobei die erhaltene α-, β- oder γ-substituierte Carbonsäure oder das Salz davon nach einem Verfahren gemäß einem der vorstehenden Ansprüche gereinigt wird.

10. Verfahren zur Racematspaltung eines Gemischs aus zwei Enantiomeren eines primären oder sekundären Amins, das folgende Schritte umfaßt:
(1) Umsetzung des racemischen Gemischs aus zwei Enantiomeren des Amins mit einem Ester, dessen Säurekomponente ein Halogen-, Stickstoff-, Sauerstoff-, Phosphor- oder Schwefelatom, gebunden an ein Kohlenstoffatom in α-, β- oder γ-Position zum Carbonylkohlenstoffatom, trägt, unter spezifischer Katalyse mit einer Hydrolase,
(2) Trennung des einen, enantioselektiv acylierten Amins (Amids) vom nicht umgesetzten, anderen Enantiomer des Amins,
(3) anschließende Hydrolyse des acylierten Amins (Amids) sowie Reinigung und Rückgewinnung einer entsprechenden α-, β-oder γ-substituierten Carbonsäure oder eines Salzes davon durch ein Verfahren wie in Anspruch 9 definiert.

11. Verfahren nach Anspruch 10, wobei als Amin Phenylethylamin eingesetzt wird.

12. Verfahren nach Anspruch 10 oder 11, wobei die gereinigte und zurückgewonnene α-, β- oder γ-substituierte Carbonsäure oder das Salz davon verestert wird und zur Umsetzung des Gemischs aus zwei Enantiomeren des Amins zurückgeführt wird.

## Claims

1. A process for purifying an α-, β- or γ-substituted carboxylic acid or a salt or an ester thereof or a mixture of two or more thereof from an aqueous solution which contains an α-, β- or γ-substituted carboxylic acid or a salt or an ester thereof or a mixture of two or more thereof and at least one polyol or at least one amino alcohol, wherein the solution is treated by electrodialysis.

2. A process as claimed in claim 1, wherein the aqueous solution additionally contains at least one cation of a strong base.

3. A process as claimed in claim 1 or 2, wherein the electrodialysis is carried out as conventional dual cycle electrodialysis, bipolar dual cycle electrodialysis or bipolar triple cycle electrodialysis or as combination of two or more thereof.

4. A process as claimed in claim 2 or 3, wherein the aqueous solution contains at least one polyol and at least one cation of a strong base and is treated firstly by conventional dual cycle electrodialysis and subsequently by bipolar triple cycle electrodialysis.

5. A process as claimed in claim 2 or 3, wherein the aqueous solution contains at least one amino alcohol and at least one cation of a strong base and is treated by bipolar dual cycle electrodialysis.

6. A process as claimed in any of the preceding claims, wherein the aqueous solution is passed through a cation exchange module during the electrodialysis or after passing through the electrodialysis.

7. A process as claimed in any of the preceding claims, wherein the α-, β- or γ-substituted carboxylic acid or a salt thereof is in the form of an aqueous solution which has been obtained in the resolution of an optically active amide to give a carboxylic acid and an optically active amine with retention of the center of chirality.

8. A process as claimed in any of the preceding claims, wherein the α-, β- or γ-substituted carboxylic acid or the salt thereof is methoxyacetic acid or a salt thereof.

9. A process for cleaving an optically active amide to a carboxylic acid or a salt thereof and an optically active amine with retention of the center of chirality, which comprises hydrolysis of the amide in the presence of at least one polyol or at least one amino alcohol and at least one cation of a strong base, the resulting α-, *β*- or γ-substituted carboxylic acid or the salt thereof being purified by a process as claimed in any of the preceding claims.

10. A process for resolving a mixture of two enantiomers of a primary or secondary amine which comprises the following steps:
(1) reacting the racemic mixture of two enantiomers of the amine with an ester whose acid component has a halogen, nitrogen, oxygen, phosphorus or sulfur atom bonded to a carbon atom in the position á, â or ã to the carbonyl carbon atom, with specific catalysis by a hydrolase,
(2) separating one amine, which is enantioselectively acylated (amide), from the other, unreacted, enantiomer of the amine,
(3) subsequently hydrolyzing the acylated amine (amide) and purifying and recovering a corresponding α-, *β*- or γ-substituted carboxylic acid or a salt thereof by a process as defined in claim 9.

11. A process as claimed in claim 10, wherein phenylethylamine is employed as amine.

12. A process as claimed in claim 10 or 11, wherein the purified and recovered α-, *β*- or γ-substituted carboxylic acid or the salt thereof is esterified and returned to the reaction of the mixture of two enantiomers of the amine.

## Revendications

1. Procédé de purification d'un acide carboxylique α, β ou γ-substitué ou d'un sel ou d'un ester de celui-ci, ou d'un mélange de deux ou plus d'entre eux, à partir d'une solution aqueuse contenant un acide carboxylique α, β ou γ-substitué ou un sel ou un ester de celui-ci, ou un mélange de deux ou plus d'entre eux et au moins un polyol ou au moins un amino-alcool, cette solution étant traitée par électrodialyse.

2. Procédé selon la revendication 1, où la solution aqueuse contient en outre au moins un cation d'une base forte.

3. Procédé selon la revendication 1 ou 2, où l'électrodialyse est mise en oeuvre sous forme d'électrodialyse à deux circuits conventionnelle, d'électrodialyse à deux circuits bipolaire ou d'électrodialyse à trois circuits bipolaire ou sous forme de combinaison de deux ou plus de celles-ci.

4. Procédé selon la revendication 2 ou 3, où la solution aqueuse contient au moins un polyol et au moins un cation d'une base forte et est d'abord traitée par électrodialyse à deux circuits conventionnelle puis par électrodialyse à trois circuits bipolaire.

5. Procédé selon la revendication 2 ou 3, où la solution aqueuse contient au moins un amino-alcool et au moins un cation d'une base forte et est traitée par électrodialyse à deux circuits bipolaire.

6. Procédé selon l'une quelconque des revendications précédentes, où la solution aqueuse est conduite à travers un module d'échange de cations, pendant l'électrodialyse ou après réalisation de l'électrodialyse.

7. Procédé selon l'une quelconque des revendications précédentes, où l'acide carboxylique α, β ou γ-substitué ou un de ses sels se trouve sous forme d'une solution aqueuse obtenue lors de la résolution du racémate d'un amide optiquement actif en un acide carboxylique et en une amine optiquement active pour obtenir le centre de chiralité.

8. Procédé selon l'une quelconque des revendications précédentes où l'acide carboxylique α, β ou γ-substitué ou son sel est l'acide méthoxyacétique ou un de ses sels.

9. Procédé de séparation d'un amide optiquement actif en un acide carboxylique ou un de ses sels et en une amine optiquement active pour obtenir le centre de chiralité, comprenant une hydrolyse de l'amide en présence d'au moins un polyol ou d'au moins un amino-alcool et d'au moins un cation d'une base forte, où l'acide carboxylique α, β ou γ-substitué obtenu ou son sel est purifié par un procédé selon l'une quelconque des revendications précédentes.

10. Procédé de résolution du racémate d'un mélange de deux énantiomères d'une amine primaire ou secondaire, comprenant les étapes suivantes :
(1) réaction du mélange racémique de deux énantiomères de l'amine avec un ester, dont le composant acide porte un atome d'halogène, d'azote, d'oxygène, de phosphore ou de soufre, lié à un atome de carbone en position α, β ou γ par rapport à l'atome de carbone du carbonyle, sous catalyse spécifique avec une hydrolase,
(2) séparation de l'amine (amide) acylée de façon énantiosélective de l'autre énantiomère de l'amine n'ayant pas réagi,
(3) hydrolyse subséquente de l'amine (amide) acylée ainsi que purification et récupération d'un acide carboxylique α, β ou γ-substitué correspondant ou d'un de ses sels, par un procédé tel que défini dans la revendication 9.

11. Procédé selon la revendication 10, où l'amine utilisée est la phényléthylamine.

12. Procédé selon la revendication 10 ou 11, où l'acide carboxylique α, β ou γ-substitué ou son sel, purifié et récupéré, est estérifié et recyclé pour la réaction du mélange des deux énantiomères de l'amine.
